(19) Europäisches Patentamt
European Patent Office
Office européen des brevets

(11) Veröffentlichungsnummer: **0 260 634 B1**

# (12) EUROPÄISCHE PATENTSCHRIFT

(45) Veröffentlichungstag der Patentschrift: **10.06.92**

(51) Int. Cl.[5]: **C07K 1/00**, C07K 1/04, C07K 5/00, C07K 7/00

(21) Anmeldenummer: **87113396.3**

(22) Anmeldetag: **14.09.87**

## (54) Verfahren zur simultanen Synthese mehrerer Peptide an fester Phase.

(30) Priorität: **17.09.86 DE 3631662**

(43) Veröffentlichungstag der Anmeldung:
**23.03.88 Patentblatt 88/12**

(45) Bekanntmachung des Hinweises auf die Patenterteilung:
**10.06.92 Patentblatt 92/24**

(84) Benannte Vertragsstaaten:
**CH DE FR GB LI NL SE**

(56) Entgegenhaltungen:
**EP-A- 0 181 491**
**GB-A- 2 158 075**

**CHEMICAL ABSTRACTS, Band 100, Nr. 23, 4. Juni 1984, Seite 619, Nr. 192243j, Columbus, Ohio, US; J.J. GORMAN: "An apparatus for simultaneous manual solid-phase synthesis of multiple peptide analogs", & ANAL. BIOCHEM. 1984, 136(2), 397-406**

**TETRAHEDRON, Band 44, Nr. 19, 1988, Seiten 6031-6040, Pergamon Press plc, GB; R. FRANK et al.: "Simultaneous multiple peptide synthesis under continuous flow conditions on cellulose paper discs as segmental solid supports"**

(73) Patentinhaber: **Gesellschaft für Biotechnologische Forschung mbH (GBF)**
**Mascheroder Weg 1**
**W-3300 Braunschweig-Stöckheim(DE)**

(72) Erfinder: **Frank, Ronald, Dr.**
**Mascheroder Weg 1**
**W-3300 Braunschweig(DE)**

(74) Vertreter: **Boeters, Hans Dietrich, Dr. et al**
**Boeters & Bauer Bereiteranger 15**
**W-8000 München 90(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Chemisch synthetisierte Peptide (Oligo- und Polypeptide) sind zu einem wichtigen Hilfsmittel in der molekularbiologischen und medizinischen Forschung geworden. Sie werden eingesetzt zur Herstellung spezifischer Antikörper für Immunaffinitätschromatographie, Identifizierung unbekannter Genprodukte und Entwicklung von Vaccinen gegen Krankheitserreger, als Peptidhormone und deren Analoga mit agonistischer oder antagonistischer Wirkung und als Modellverbindungen in Proteinstrukturuntersuchungen.
Peptide sind Oligo- bzw. Polymere (n bis etwa 150), aufgebaut durch Verknüpfung von Aminosäuren über Amidbindungen (Peptidbindungen).

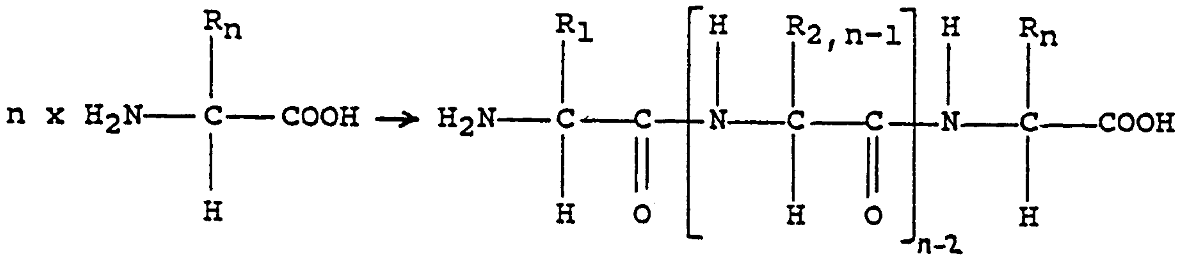

Aminosäure Peptid

Im vorliegenden Zusammenhang werden unter Peptiden auch solche verstanden, die außer den 20 natürlichen L-Amionosäuren ebenso D-Aminosäuren, seltene und nicht natürliche Aminosäuren, als auch chemisch modifizierte und synthetische Aminosäuren enthalten (R ist beliebig). Die chemische Synthese von Peptiden mit vorgegebener Sequenz der Aminosäuren verläuft schrittweise durch Verknüpfung von geeignet geschützten Aminosäure-, Di-, oder Oligopeptidbausteinen. Verschiedene Synthesemethoden, die sich in der Art der Schutzgruppen und Chemie der Knüpfung der Amidbindung unterscheiden, gehören zum Stand der Technik. Eine Übersicht ist gegeben in:

(1) R.B. Merrifield, J. Am. Chem. Soc., 85 (1963) 2 149.

(2) E. Wünsch et al. in Houben-Weyl: Methoden der organischen Chemie, 4. Auflage, Band 15 (E. Müller, Herausgeber) Thieme, Stuttgart, 1974.

(3) G. Barany, R.B. Merrifield in The Peptides, Vol. 2 (E. Gross, J. Meienhofer, Eds) Academic Press, New York, 1979, p. 1.

Schnelle Synthesen werden nach dem von Merrifield entwickelten Prinzip an fester Phase durchgeführt, wobei die wachsenden Ketten des Oligomeren üblicherweise mit dem Ende, das nicht verlängert wird, chemisch an eine feste Phase in Form eines makroskopisch festen Trägermaterials gebunden sind. Die Kettenverlängerungsreaktionen bzw. Verknüpfungsreaktionen werden dann an der Oberfläche des Trägermaterials durchgeführt, wobei das Reaktionsprodukt am Träger gebunden bleibt und Reaktionsüberschüsse und Reaktionskomponenten durch Waschen entfernt werden.

Bisher ist eine Vielzahl von Trägermaterialien für derartige Synthesen eingesetzt worden, wobei Trägermaterial und Synthesemethode aufeinander abgestimmt sein müssen; vgl. locus citatus und darin angeführte Literatur. Der Fachmann ist mit dieser Abstimmung vertraut. So werden Peptide bisher im allgemeinen einzeln jeweils an einer kleinen Menge Granulat synthetisiert; vgl. beispielsweise Rietschoten et al., in: Peptides 1974, Proc. 13th Europ. Peptide Symp., Seiten 113-116, John Wiley & Sons. Es ist auch bekannt, Peptide einzeln an Kunststofffolien zu synthetisieren; vgl. beispielsweise DE-OS 1 593 880.4. Sollen nach diesem Stand der Technik n Peptide mit einer Kettenlänge von m Aminosäureeinheiten hergestellt werden, so werden in der Regel n x m Syntheseschritte vorgenommen. Es ist allerdings auch in Säckchen verpacktes Granulat vorgeschlagen worden, die zu Gruppen zusammengefaßt werden können; Houghten, Proc. Natl. Acad. Sci. USA, 82 (1985) 5131.

Aufgabe der Erfindung ist es, ein Verfahren zur simultanen Synthese mehrerer Peptide an fester Phase vorzusehen, bei dem man ein, zwei oder mehr Träger aus einem Flachmaterial verwendet, das für die verwendeten flüssigen Medien durchlässig ist und eine zum Einsatz in Säulenreaktoren ausreichende mechanische Stabilität und geeignete Form aufweist.

Diese Aufgabe wird erfindungsgemäß durch ein Verfahren zur simultanen Synthese mehrerer Peptide an fester Phase gelöst, bei dem man als feste Phase ein, zwei oder mehr Träger aus einem für die verwendeten flüssigen Medien durchlässigen Flachmaterial aus Zellulose oder Papier (jeweils gegebenenfalls faserverstärkt) verwendet und in an sich bekannter Weise

(a) die jeweils 1. Aminosäuren als Startbausteine gruppenweise mit den in geeigneter Weise funktionalisierten Trägersegmenten verknüpft, wobei jede Trägergruppe mindestens ein Trägersegment umfaßt, und die Trägersegmente mit von Gruppe zu Gruppe unterschiedlichen Startbausteinen verknüpft (vgl. Schema 1), und

(b) die aus (a) resultierenden beladenen Trägersegmente gruppenweise gemäß den Sequenzen der zu synthetisierenden Peptide mit den entsprechenden Aminosäurebausteinen umsetzt (vgl. Schema 1), wobei

(c) gemäß der gewählten Synthesemethode der Stufe (b) ein Entschützungsschritt vorausgehen und auf die Stufe (b) ein Blockierungsschritt folgen kann.

Zellulose und Papier sind keine chemisch inerten Materialien une weisen keine homogene Porenverteilung auf; vgl. beispielsweise Amarnath & Broom, Chem. Rev., 77 (1977) 103. Nicht umsonst hat der Stand der Technik bisher Kunststoffträger verwendet, die chemisch speziell auf die gewählte Peptidsynthese abgestimmt sind. Es war daher überraschend, daß Zellulose und Papier die Peptidsynthese nicht beeinträchtigen, so daß ihre Vorteile genutzt werden können, nämlich ihre leichte Verfügbarkeit, ihre mechanische Stabilität und ihre gute Durchlässigkeit für die eingesetzten flüssigen Medien.

Unter einem erfindungsgemäß verwendbaren Flachmaterial wird ein Material verstanden, bei dem eine der drei zueinander senkrecht räumlichen Dimensionen wesentlich kleiner als die beiden anderen Dimensionen ist. Diese Träger aus Flachmaterial werden mit beliebiger räumlich begrenzter Form als Segmente verwendet, beispielsweise in Form von Blättern oder Streifen. Beispiele für derartige Flachmaterialien sind Filtrationspapier und mit Glasfasern verstärktes Filtrationspapier. Das Flachmaterial kann per se zum Verknüpfen mit dem 1. Aminosäurebaustein (Startbaustein) geeignet sein oder es kann in einer dem Fachmann z.B. aus dem Stand der Technik geläufigen Methode dafür modifiziert worden sein.

Gemäß einer bevorzugten Ausführungsform ist das verwendete Flachmaterial für das flüssige Reaktionsmedium und/oder für die darin enthaltenen Reaktionkomponenten durchlässig.

Die erfindungsgemäße Verwendung von Trägern aus Flachmaterial erlaubt es, daß mehrere Trägersegmente gleichzeitig in ein und demselben Reaktionsgefäß mit ein und demselben Aminosäurebaustein umgesetzt und danach wieder eindeutig und ohne jede Kontamination voneinander getrennt werden können.

Jedes Peptid wird jeweils an ein oder mehreren Segmenten einer Trägergruppe synthetisiert. Dazu werden die ersten Aminosäuren der zu synthetisierenden Peptidketten gemäß einer an sich bekannten und auf das jeweilige Trägermaterial abgestimmten Methode chemisch an den Träger gebunden. Danach werden gegebenenfalls restliche reaktive Funktionen der mit den Startbausteinen verknüpften Trägersegmente blockiert und die Startbausteine terminal entschützt. Trägergruppen, an deren Segmente jeweils der gleiche Synthesebaustein geknüpft werden soll, werden zusammen in ein und demselben Reaktionsgefäß umgesetzt.Die Verknüpfungsreaktionen werden nach der jeweils angewandten Synthesemethode durchgeführt. Nach der Verknüpfung kann wieder blockiert werden und terminal entschützt werden. Danach folgen die nächsten Verknüpfungszyklen, wozu die Trägersegmente gruppenweise entsprechend der zu synthetisierenden Sequenzen mit den Aminosäurebausteinen umgesetzt werden. Ein möglicher Syntheseablauf ist beispielhaft in Schema 1 wiedergegeben.Nach dem letzten Verknüpfungsschritt könn en alle Trägersegmente getrennt, die Peptide von den Trägersegmenten abgespalten, entschützt und isoliert werden.

An Stelle der geeignet geschützten Aminosäurebausteine können auch geeignet geschützte Di- oder Oligopeptide für den Aufnau der Peptide eingesetzt werden. Die Syntheseschritte können nach Stand der Technik sowohl in schüttelbaren Reaktionsgefäßen als auch in Säulenreaktoren (wie beispielsweise von Dryland & Sheppard, J. Chem. Soc. Perkin Trans., I (1986) 125, vorgeschlagen) von manuell zu bedienenden oder automatisierten Synthesevorrichtungen durchgeführt werden.

Nach dem erfindungsgemäßen Verfahren reduziert sich die Anzahl der Syntheseschritte für die Herstellung von mehreren Peptiden entsprechend der durch die Sequenzen gegebenen Möglichkeit, verschiedene Trägergruppen (für unterschiedliche Peptidsequenzen) in möglichst vielen Verlängerungszyklen gemeinsam umzusetzen. Im Beispiel aus Schema 1 reduziert sich die Zahl der Reaktionszyklen von 7 x 10 = 70 auf 15. Besonders vorteilhaft ist das Verfahren für die Herstellung einer Anzahl von Sequenzvarianten, wobei sich die Peptidsequenzen in nur wenigen Positionen voneinander unterscheiden.

Für die errindungsgemäße Peptidsynthese können bekannte Synthesemethoden eingesetzt werden. Nachstehend wird die Erfindung durch ein Beispiel näher erläutert. In Schema 2 ist gezeigt, wie ein Trägermaterial aus Cellulose (beispielsweise Filterpapier) mit Monomethoxytrityl-6/p-oxymethyl-(phenoxy)-/hexansäure und MSNT als Kondensationsmittel verestert wird. Nach Blockierung restlicher zugänglicher OH-Gruppen der Cellulose wird die Monomethoxytritylgruppe mit Dichloressigsäure abgespalten und an die entstehenden freien p-Hydroxymethylgruppen der 1. Aminosäurebaustein über eine Esterbindung geknüpft. Eine Blockierung restlicher, nicht umgesetzter p-Hydroxymethylgruppen kann folgen. Danach wird die $NH_2$ Gruppe des 1. Aminosäurebausteins entschützt und mit der Carboxylgruppe des 2. Aminosäurebausteins

verknüpft, wonach verbliebene freie $NH_2$-Gruppen blockiert werden können. Durch die zyklische Abfolge von Entschützung der endständigen $NH_2$-Gruppen , Anknüpfung des nächsten Aminosäurebausteins und gegebenenfalls Blockierung nicht umgesetzter $NH_2$-Gruppen wird die Peptidkette aufgebaut. Für die Verknüpfungsreaktionen wurden die geeigent geschützten Aminosäureanhydride bzw. p-Nitrophenylester verwendet. Die Fluorenylmethoxycarbonylgruppe wurde als temporäre $NH_2$-Schutzgruppe gewählt, die mit 20% Piperidin in Dimethylformamid jeweils abgespalten wurde. Es ließen sich Peptide mit 7 und mehr Aminosäuren in der Kette herstellen. Die Ausbeuten waren mit dem Stand der Technik vergleichbar.

Erklärung zu Schema 1

| | |
|---|---|
| $AS_n$ | = Aminosäure, gleicher Index = gleiche Aminosäure |
| $Tr_n$ | = Trägersegmente der Gruppe n für Sequenz n |
| ZN | = n-ter Verknüpfungszyklus |

Erklärung zu Schema 2

| | |
|---|---|
| P | = Trägermaterial (Polymer, in diesem Fall Cellulose) |
| MeOTr | = p-Monomethoxytrityl- |
| MSNT | = 2,4,6 -Mesitylen-3-nitro-1,2,4 -triazolid |
| Ac | = Acetyl- |
| DCC | = Dicyclohexylcarbodiimid |
| DMAP | = 4-Dimethylaminopyridin |
| DIPEA | = Diisopropylethylamin |
| $R_n$ | = Aminosäureseitenrest |
| Fmoc | = Fluorenylmethoxycarbonyl- |
| DMF | = Dimethylformamid |
| DCA | = Dichloressigsäure |

Schema 1

| | | | | | | | |
|---|---|---|---|---|---|---|---|
| Sequenz 1 | Z14 $AS_{14}$ - | Z10 $AS_{10}$ - | Z9 $AS_8$ - | Z7 $AS_7$ - | Z4 $AS_4$ - | Z3 $AS_3$ - | Z1 $AS_1$ |
| Sequenz 2 | $AS_{14}$ - | $AS_{10}$ - | $AS_8$ - | $AS_7$ - | Z5 $AS_5$ - | $AS_3$ - | $AS_1$ |
| Sequenz 3 | $AS_{14}$ - | Z11 $AS_{11}$ - | Z8 $AS_9$ - | $AS_7$ - | Z6 $AS_6$ - | $AS_3$ - | $AS_1$ |
| Sequenz 4 | Z13 $AS_{13}$ - | Z9 $AS_8$ - | $AS_9$ - | $AS_7$ - | Z5 $AS_5$ - | $AS_3$ - | $AS_1$ |
| Sequenz 5 | $AS_{13}$ - | Z10 $AS_{10}$ - | $AS_9$ - | $AS_7$ - | Z6 $AS_6$ - | $AS_3$ - | $AS_1$ |
| Sequenz 6 | $AS_{13}$ - | Z12 $AS_{12}$ - | $AS_9$ - | $AS_7$ - | Z4 $AS_4$ - | $AS_3$ - | Z2 $AS_2$ |
| Sequenz 7 | Z15 $AS_9$ - | Z10 $AS_{10}$ - | Z9 $AS_8$ - | $AS_7$ - | Z5 $AS_5$ - | $AS_3$ - | $AS_2$ |
| Sequenz 8 | $AS_9$ - | $AS_{10}$ - | Z8 $AS_9$ - | $AS_7$ - | Z6 $AS_6$ - | $AS_3$ - | $AS_2$ |
| Sequenz 9 | $AS_9$ - | Z13 $AS_{13}$ - | $AS_9$ - | $AS_7$ - | $AS_6$ - | $AS_3$ - | $AS_2$ |
| Sequenz 10 | Z14 $AS_{14}$ - | Z9 $AS_8$ - | $AS_9$ - | $AS_7$ - | Z4 $AS_4$ - | $AS_3$ - | $AS_2$ |

1. Zyklus : $Tr_{1-5}$ verknüpfen mit $AS_1$ (Beladung)
2. Zyklus : $Tr_{6-10}$ verknüpfen mit $AS_2$ (Beladung)
3. Zyklus : $Tr_{1-10}$ verknüpfen mit $AS_3$
4. Zyklus : $Tr_{1,6,10}$ verknüpfen mit $AS_4$
5. Zyklus : $Tr_{2,4,7}$ verknüpfen mit $AS_5$
6. Zyklus : $Tr_{3,5,8,9}$ verknüpfen mit $AS_6$
7. Zyklus : $Tr_{1-10}$ verknüpfen mit $AS_7$
8. Zyklus : $Tr_{3,4,5,6,8,9,10}$ verknüpfen mit $AS_9$
9. Zyklus : $Tr_{1,2,4,7,10}$ verknüpfen mit $AS_8$
10. Zyklus: $Tr_{1,2,5,7,8}$ verknüpfen mit $AS_{10}$
11. Zyklus: $Tr_3$ verknüpfen mit $AS_{11}$
12. Zyklus: $Tr_6$ verknüpfen mit $AS_{12}$
13. Zyklus: $Tr_{4,5,6,9}$ verknüpfen mit $AS_{13}$
14. Zyklus: $Tr_{1,2,3,10}$ verknüpfen mit $AS_{14}$
15. Zyklus: $Tr_{7,8,9}$ verknüpfen mit $AS_9$

Schema 2 Synthese von Peptiden an Cellulose als "fester Phase"

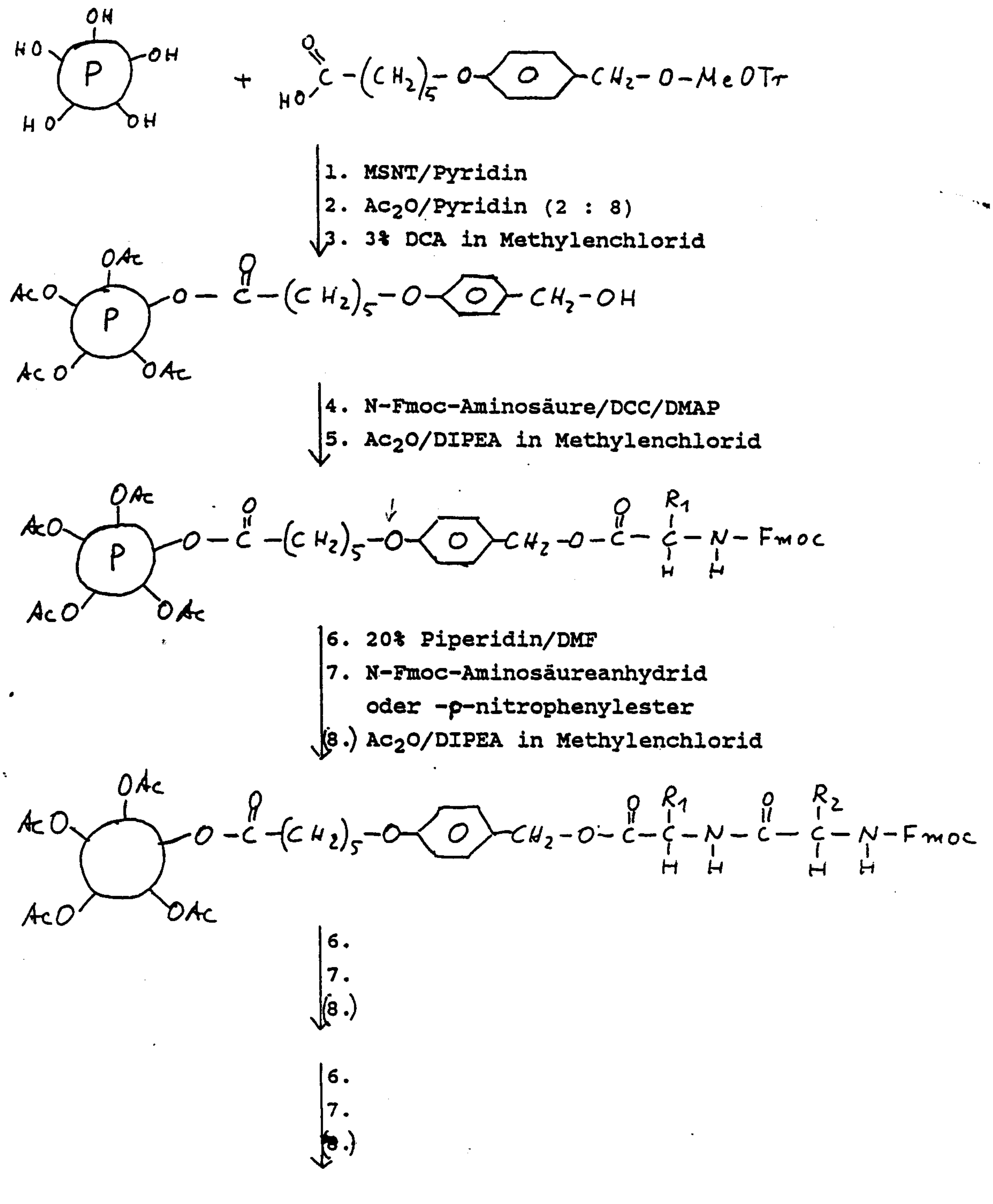


## Patentansprüche

1. Verfahren zur simultanen Synthese mehrerer Peptide an fester Phase, dadurch **gekennzeichnet,** daß man als feste Phase ein, zwei oder mehr Träger aus einem für die verwendeten flüssigen Medien durchlässigen Flachmaterial aus Cellulose oder Papier (jeweils ggf. faserverstärkt) verwendet und in an sich bekannter Weise

   (a) die jeweils 1. Aminosäuren als Startbausteine gruppenweise mit den in geeigneter Weise funktionalisierten Trägersegmenten verknüpft, wobei jede Trägergruppe mindestens ein Trägersegment umfaßt, und die Trägersegmente mit von Gruppe zu Gruppe unterschiedlichen Startbausteinen verknüpft, und
(b) die aus (a) resultierenden beladenen Trägersegmente gruppenweise gemäß den Sequenzen der zu synthetisierenden Peptide mit den entsprechenden Aminosäurebausteinen umsetzt, wobei
(c) gemäß der gewählten Synthesemethode der Stufe (b) ein Entschützungsschritt vorausgehen und auf die Stufe (b) ein Blockierungsschritt folgen kann.

**2.** Verfahren nach Anspruch 1, dadurch **gekennzeichnet,** daß man das Verfahren in einem oder mehreren Säulenreaktor durchführt.

**3.** Verfahren nach einem der vorhergehenden Ansprüche, dadurch **gekennzeichnet,** daß die Trägersegmente quer zur Strömungsrichtung angeordnet werden und jeweils die gesamte innere Querschnittsfläche des Säulenreaktors einnehmen.

## Claims

**1.** Process for the simultaneous synthesis of a plurality of peptides on a solid phase, characterized in that as solid phase one, two or more substrates are used comprising a flat material of cellulose or paper (possibly each fibre-reinforced) permeable to the fluid media used and in a manner known per se
(a) the respective 1st amino acids are linked as start modules in groups to the substrate segments functionalized in suitable manner, each substrate group including at least one substrate segment, and the substrate segments are linked with start modules differing from group to group, and
(b) the charged substrate segments resulting from (a) are reacted in groups with the corresponding amino acid modules in accordance with the sequences of the peptides to be synthesized,
(c) and according to the selected synthesis method the stage (b) may be preceded by a deprotection step and followed by a blocking step.

**2.** Process according to claim 1, characterized in that the process is carried out in one or several column reactors.

**3.** Process according to any one of the preceding claims, characterized in that the substrate segments are arranged transversely of the flow direction and in each case take up the entire inner cross-sectional area of the column reactor.

## Revendications

**1.** Procédé de synthèse simultanée de plusieurs peptides sur une phase solide, caractérisé en ce qu'on utilise comme phase solide, un, deux ou plusieurs supports constitués d'une matière planiforme, perméable aux milieux liquides utilisés, de cellulose ou de papier (renforcé de fibres le cas échéant), et en ce que, d'une manière connue en soi,
(a) on lie par groupes les aminoacides n° 1 comme éléments constitutifs de départ aux segments de support fonctionnalisés d'une manière appropriée, chaque groupe de support comprenant au moins un segment de support, et on lie les segments de support à des éléments constitutifs de départ différents d'un groupe à l'autre, et
(b) on fait réagir les segments de support chargés obtenus en (a) par groupes conformément aux séquences des peptides à synthétiser avec les aminoacides constitutifs correspondants,
(c) une étape de déprotection pouvant précéder l'étape (b) et une étape de blocage pouvant suivre l'étape (b) selon le procédé de synthèse choisi.

**2.** Procédé selon la revendication 1, caractérisé en ce qu'on effectue le procédé dans un ou plusieurs réacteurs à colonnes.

**3.** Procédé selon l'une des revendications précédentes, caractérisé en ce que les segments de support sont disposés transversalement à la direction de l'écoulement, et occupent chaque fois la totalité de la surface de la section transversale interne du réacteur à colonnes.